# EUROPEAN PATENT SPECIFICATION

(11) **EP 4 017 378 B1**
(45) Date of publication and mention of the grant of the patent: **27.05.2026**
(21) Application number: 20754776.1
(22) Date of filing: 19.08.2020
(51) Int. Cl.: A61B 17/04, A61B 17/00

(54) **IMPLANTABLE SUSPENSION DEVICE AND ASSEMBLY DEVICE**
IMPLANTIERBARE AUFHÄNGUNGSVORRICHTUNG UND MONTAGEVORRICHTUNG
DISPOSITIF DE SUSPENSION IMPLANTABLE ET DISPOSITIF D'ASSEMBLAGE

(30) Priority: 19.08.2019 EP 19192320
(43) Date of publication of application: 29.06.2022
(73) Proprietor: ZuriMED Technologies AG, 8008 Zürich (CH)
(72) Inventor: LI, Xiang, 8126 Zumikon (CH); BACHMANN, Elias, 8706 Meilen (CH); SCHUBERT, Jan, 8050 Zürich (CH)
(74) Representative: Schulz Junghans Patentanwälte PartGmbB
(86) International application number: PCT/EP2020/073191
(87) International publication number: WO 2021/032778

(56) References cited:
- EP-A1- 2 581 047
- EP-A2- 2 238 944
- WO-A2-2012/154922
- US-A1- 2010 256 677
- US-A1- 2018 360 440
- US-A1- 2019 099 258
- US-B2- 9 101 461
- US-B2- 9 549 811

## Description

The disclosure relates to a suspension device which is implantable into the human or animal body, particularly for connecting soft tissue (i.e. tendon or ligament) to bone, an assembly device for assembling the implantable suspension device and a method for assembling the implantable suspension device.

Implantable suspension devices, also termed cortical fixation devices (CFD), are widely used to fix soft tissue (i.e. tendon or ligament grafts) to bone during surgical procedures such as anterior cruciate ligament (ACL) reconstruction, Achilles tendon repair, biceps tendon repair, meniscus root repair or rotator cuff repair. Such devices commonly comprise a suture element used to connect the soft tissue to the device and a suspension plate or button attaching to the bone.

In the known implantable suspension devices according to the prior art, the suture element forms a closed loop, which requires the soft tissue to pass across the loop and fold in order to be connected to the suspension device, resulting in the closed loop contacting the middle part of the folded implant.

However, the other side of the folded graft is open, requiring the use of an interference screw to press-fit the implant into a bone tunnel.

Additionally, in many cases, it is not possible to fit the folded implant to the closed loop for cortical fixation.

Particularly, US2018360440A1 discloses a method of reinforcing a biological construct comprising attaching a suture loop/needle construct to a reinforcement material and stitching the reinforcement material to a biological construct to form a reinforced biological construct. The reinforcement material is attached to the suture loop/needle construct prior to approximating the reinforcement material to the biological construct.

Therefore, the objective underlying the present disclosure is to provide an implantable suspension device which is improved in view of the above-stated disadvantages of the prior art.

The invention relates to an implantable suspension device and is defined by the features of independent claim 1. Embodiments are given in the dependent claims.

A first aspect of the disclosure relates to an implantable suspension device for fixing an elongated flexible implant (e.g. a ligament or tendon graft) or tissue (such as bone tissue) in a desired position, wherein the implantable suspension device comprises a suspension plate and a suture element engaging with the suspension plate, wherein the suture element comprises a first end section extending from the suspension plate to a first end, particularly a free first end, of the suture element, and a second end section extending from the suspension plate to a second end, particularly a free second end, of the suture element, wherein the second end section comprises a connecting section configured to be connected to the implant or to a medical textile, particularly by stitching or felting. Further, said suture element comprises or consists of a plurality of fibers, wherein the fibers are separated from each other in the connecting section, wherein the connecting section forms a fan-like structure extending in a plane parallel to an extension direction of the suture element.

In particular, the implantable suspension device (also termed cortical fixation device) is suitable for fixing soft tissue, e.g. a tendon or ligament graft, to a bone. Medical applications of such suspension devices include anterior cruciate ligament (ACL) reconstruction, Achilles tendon repair, biceps tendon repair, meniscus root repair or rotator cuff repair. In particular, in case of an ACL reconstruction, the suspension plate, particularly its rear side, is configured to butt against the tibia or femur of the patient, wherein particularly the second end section extends through a bore hole drilled into the tibia or femur.

In the context of the present specification, the term suspension plate (also termed button) designates a member being configured to secure an implant, particularly a tendon or ligament graft, at its desired location, particularly in a bone tunnel. In particular, in its desired location, the suspension plate abuts a bone adjacent to (outside of) a bone tunnel harboring at least a section of the implant.

In the context of the present specification, the term suture element designates an elongated and flexible member (such as a thread, a ribbon or a wire) configured to be connected to tissue by stitching. The suture element may be from any suitable material, e.g. natural or synthetic fabric, polymers or metal.

The expression "a suture element engaging with the suspension plate" means that the suture element is mechanically connected to the suspension plate, e.g. by insertion of the suture element into at least one through-hole of the suspension plate.

In contrast to implantable suspension devices of the prior art which use a closed suture loop for connection to the middle portion of a folded implant, the suspension device according to the present disclosure allows to fix one end of an unfolded implant to the connecting section, such that the other end of the implant is free, particularly for connecting a further suspension device. Thus, the suspension device according to the disclosure can be more easily connected to an implant, and the resulting assembly is more versatile compared to the prior art.

In certain embodiments, the connecting section is positioned at the second end of the suture element.

In certain embodiments, the suture element forms a first locking loop for pressing the first end section of the suture element against the suspension plate and thereby locking the first end section with respect to the suspension plate. In particular, the first locking loop is configured to receive the first end section, such that the first end section extends through the first locking loop.

The locking loop provides a self-locking mechanism eliminating the need for separate fixing means to attach the suture element to the suspension plate.

In certain embodiments, the second end section comprises an adjustable first suspension section between the suspension plate and the connecting section, wherein the first suspension section is configured to be shortened by pulling on the first end section of the suture element, wherein particularly the first end section protrudes out of the first locking loop.

Using the first suspension section, the length of the first suspension section may be adjusted to the needs of the individual patient in an easy manner, particularly adjusted to the length of the bone tunnel.

In certain embodiments, the suspension plate comprises a front side and a rear side, which rear side faces away from the front side.

In certain embodiments, the suspension plate comprises a plurality of through-holes, each through-hole extending from the front side to the rear side of the suspension plate, wherein the suture element extends through the through-holes.

In certain embodiments, the plurality of through-holes is formed by at least three through-holes. In certain embodiments, the plurality of through-holes is formed by 3, 4 or 6 through-holes.

In certain embodiments, the suture element extends through a first-through-hole of the plurality of through-holes from the rear side to the front side of the suspension plate, with the first end ahead, particularly such that the adjustable first suspension section is formed on the rear side of the suspension plate.

In certain embodiments, the suture element further extends through a second through-hole of the plurality of through-holes from the front side to the rear side of the suspension plate, with the first end ahead, particularly such that the first locking loop is formed on the front side of the suspension plate.

In certain embodiments, the suture element further extends through a third through-hole of the plurality of through-holes from the rear side to the front side of the suspension plate with the first end ahead. In particular, the first end section of the suture element further extends through the first locking loop for clamping the first end section to the front side of the suspension plate by means of the first locking loop with a locking force.

In certain embodiments, the suture element further extends through a fourth through-hole of the plurality of through-holes from the front side to the rear side of the suspension plate with the first end ahead.

In certain embodiments, the suture element further extends through a fourth through-hole of the plurality of through-holes from the rear side to the front side of the suspension plate with the second end ahead, particularly such that the suture element forms a second suspension section between the connecting section and the suspension plate.

In certain embodiments, the suture element further extends through a fifth through-hole of the plurality of through-holes from the front side to the rear side of the suspension plate with the second end ahead, particularly such that a second locking loop for pressing the second end section against the suspension plate and thereby locking the second end section with respect to the suspension plate is formed. In particular, the second locking loop is configured to receive the second end section, such that the second end section extends through the second locking loop.

In certain embodiments, the suture element further extends through a sixth through-hole of the plurality of through-holes from the rear side to the front side of the suspension plate with the second end ahead, wherein particularly the second end section of the suture element further extends through the second locking loop for clamping the second end section of the suture element to the front side of the suspension plate by means of the second locking loop with a locking force.

In certain embodiments, the second end section comprises an adjustable second suspension section between the suspension plate and the connecting section, wherein the second suspension section is configured to be shortened by pulling on the second end section of the suture element, particularly the second end of the suture element. In particular, the second end section protrudes out of the second locking loop.

In certain embodiments, the suspension plate is connected to or integrally formed with a screw or an anchor configured to be inserted into a bone. Thereby, the suspension plate can be tightly fixed to a bone where required, while being able to easily connect soft tissue to the suspension plate, and particularly adjust the length of the first and/or second suspension section.

In certain embodiments, the suture element comprises a needle attached to the suture element, particularly to the first end or the second end. Using the needle, the connecting section may be connected to an implant without further tools.

In certain embodiments, the suture element is braided from an ultra-high molecular weight polyethylene. In certain embodiments, the suture element is co-braided from an ultra-high molecular weight polyethylene and polypropylene, polyester or polyamide.

In certain embodiments, the suspension plate, particularly the front side and/or the rear side of the suspension plate, has a surface roughness of at least 0.6 µm.

According to the disclosure, the suture element comprises or consists of a plurality of fibers (particularly braided or twisted fibers), wherein the fibers are separated from each other in the connecting section, particularly wherein each of the separated fibers comprises a fiber end or a fiber loop positioned at the second end of the suture element. Such fibers can be advantageously connected to a medical textile or an implant by felting (also termed "needle felting"), i.e., repeatedly advancing a needle comprising at least one barb through the connecting section and the medical textile or implant.

Furthermore, according to the disclosure, the fibers are separated from each other in the connecting section. That means that, as opposed to the remaining suture element, the fibers in the connecting section are not connected, particularly braided (i.e. entangled), twisted, knitted or felted, with neighboring fibers. Of course, the fibers may still contact neighboring fibers in the connecting section.

By means of the separated fibers of the connecting section, which are spread over a larger area than in the main section of the medical implant, pressure can be advantageously distributed over a larger area when the connecting section is connected to soft tissue thereby improving the stability of the mechanical connection. At the same time, the good tensile strength of the suspension device is retained due to the braided or twisted suture element.

According to the disclosure, the connecting section forms a fan-like structure extending in a plane parallel to an extension direction of the suture element. This advantageously increases the area of the connecting section, thereby improving the connection strength.

A second aspect of the disclosure relates to a medical implant comprising the implantable suspension device according to the first aspect and a medical textile, particularly comprising or consisting of a felt material, wherein the connecting section of the suture element is connected to the medical textile, and wherein the medical textile is configured to be connected to the elongated flexible implant. Such a medical textile advantageously improves the connection of the suture element to the implant.

In certain embodiments, the connecting section is connected to the medical textile by stitches. In certain embodiments, the suture element comprises or consists of a plurality of fibers, wherein the fibers are separated from each other in the connecting section, and wherein the separated fibers of the connecting section extend into and/or through the medical textile to connect the suture element of the implantable suspension device to the medical textile. This type of connection can be particularly achieved by advancing a needle having at least one barb repeatedly through the connecting section and the medical textile, resulting in a very strong connection using a fast and simple procedure.

In certain embodiments, the medical textile extends along a plane.

In certain embodiments, the medical textile comprises a flat shape.

In certain embodiments, the connecting section of the suture element forms a fan-like structure extending in a plane parallel to an extension direction of the suture element, wherein the suture element is connected to the medical textile, such that the first medical implant extends from the medical textile parallel to the plane of the medical textile. This further increases the area of the connecting section, contributing to the connection strength.

In certain embodiments, the medical textile comprises a tubular shape, wherein the medical textile comprises an inner surface configured to be connected to the elongated flexible implant. This allows an especially tight connection to implants having an elongated shape, such as tendons.

Further features and advantages of the disclosure shall be described by means of detailed descriptions of embodiments with reference to the Figures, wherein
- Fig. 1: shows the assembly of an implantable suspension device not forming part of the invention;
- Fig. 2: shows the assembly of an implantable suspension device not forming part of the invention;
- Fig. 3: shows the assembly of an implantable suspension device not forming part of the invention;
- Fig. 4: shows a further embodiment of the implantable suspension device not forming part of the invention, where the suspension plate is integrally formed with a bone screw;
- Fig. 5: shows a further embodiment of the implantable suspension device not forming part of the invention, where the suspension plate is integrally formed with a bone anchor;
- Fig. 6: schematically shows the threading of the suture element through the through-holes of the suspension plate by means of a looped guiding rope not forming part of the invention;
- Fig. 7: shows a hollow guiding rope with a funnel structure for engaging the suture element with the suspension plate of the suspension device not forming part of the invention;
- Fig. 8: shows the assembly of the suture element with the suspension plate using an assembly device not forming part of the invention;
- Fig. 9: shows an assembly device not forming part of the invention including a tool for implanting the suspension device comprising a handle and a guide bar;
- Fig. 10: shows details of the assembly device according to Fig. 9;
- Fig. 11: shows the disassembly of the assembly device according to Fig. 9 after engagement of the suture element with the suspension plate;
- Fig. 12: shows an assembly device not forming part of the invention which is separable into a handle part and a tip part;
- Fig. 13: shows details of the tip part according to Fig. 12;
- Fig. 14: shows a further embodiment of an assembly device not forming part of the invention comprising a first part and a second part which can be rotated with respect to each other;
- Fig. 15: shows details of the assembly device according to Fig. 14;
- Fig. 16: shows the assembly of a medical implant according to an embodiment of the invention;
- Fig. 17: shows the assembly of a medical implant according to a further embodiment of the disclosure.

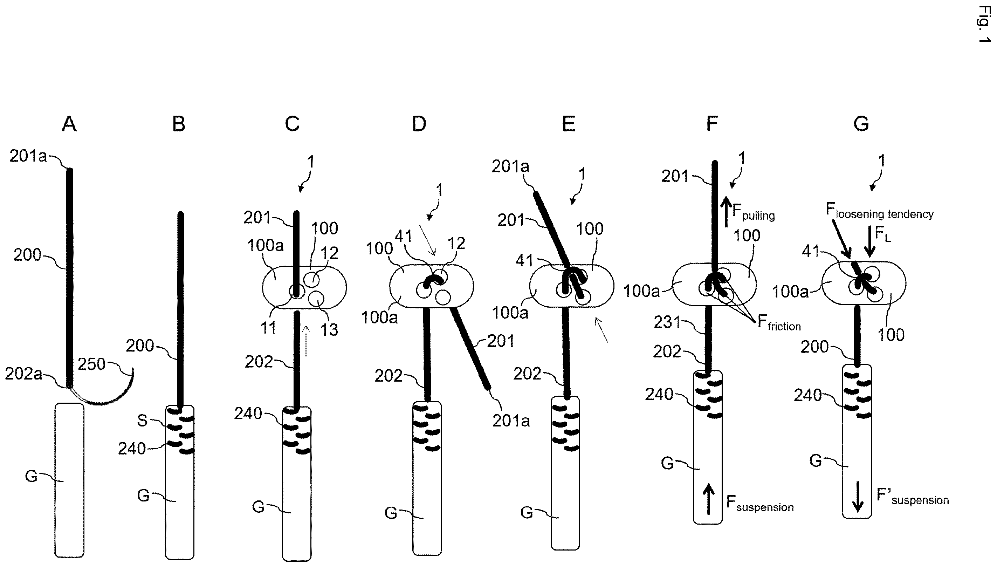

Fig. 1A-G show assembly steps of a first embodiment of the implantable suspension device 1 according to the invention. The implantable suspension device 1 comprises a suspension plate 100 and a suture element 200.

As shown in Fig. 1 A, the suture element 200 comprises a first free end 201a and a second free end 202a. A connecting section 240 disposed at the second end 202a of the suture element 200 is connected to an implant G, particularly a soft tissue such as a tendon or ligament, by stitches S (Fig. 1B). For example, the connecting section 240 of the suture element 200 may be connected to the implant G by means of a needle 250 attached to the second end 202a of the suture element 200 as shown in Fig. 1A. After stitching the connecting section 240 to the implant G, the needle 250 may be removed by cutting the suture element 200.

The first end 201a is then advanced through a first through-hole 11 of the suspension plate 100 from a rear side 100b (not shown in Fig. 1) to a front side 100a (Fig. 1C). In this manner, a first end section 201 protruding from the first through-hole 11 on the front side 100a of the suspension plate 100 and a second end section 202 disposed between the implant G and the rear side 100b of the suspension plate 100 are formed.

Subsequently, the first end 201a of the suture element 200 is inserted into a second through hole 12 of the suspension plate 100 from the front side 108 to the rear side 100 B (Fig. 1D), such that a first locking loop 41 is formed on the front side 100a of the suspension plate 100 between the first through-hole 11 and the second through-hole 12.

In the next step which is shown in Fig. 1E, the first end 201a of the suture element 200 is inserted into a third through-hole 13 from the rear side 200b to the front side 100a of the suspension plate 100 and advanced through the first locking loop 41.

The final assembly with a tightened first locking loop 41 pressing the first end section 201 against the front side 100a of the suspension plate 100 is shown in Fig. 1F.

Furthermore, a first suspension section 231 is formed between the rear side 100b of the suspension plate 100 and the connecting section 240 stitched to the implant G (Fig. 1F). The length of the first suspension section 231 may be adjusted by pulling on the first end section 201 of the suture element 200 protruding from the first locking loop 41 on the front side 100a of the suspension plate 100 with a pulling force **F_{pulling}** (Fig. 1F). During the pulling process, the pulling force **F_{pulling}** equals the sum of the suspension force Fₛᵤₛₚₑₙₛᵢₒₙ acting on the implant G via the first suspension section 231 and the friction force F_{friction} **(F_{pulling}** = **F**ₛᵤₛₚₑₙₛᵢₒₙ + **F**_{friction}).

As illustrated in Fig. 1 G, when the first end section 201 is released, the first end section 201 is locked by the first locking loop 41 from loosening. In this situation, the suspension force F'ₛᵤₛₚₑₙₛᵢₒₙ equals the sum of the loosening tendency force F_{loosening tenancy} and the friction force F'friction (F'ₛᵤₛₚₑₙₛᵢₒₙ = F_{loosening} tendency + F'_{friction}). The locking force F_{locking} equals the friction coefficient µ_{friction} times the suspension force F'ₛᵤₛₚₑₙₛᵢₒₙ. **(F_{locking}** = **µ_{friction}** x **F**'ₛᵤₛₚₑₙₛᵢₒₙ). Because the locking force F_{locking} is higher than the loosening tendency force F_{loosening tendency}, **(F_{locking} > F_{loosening} t_{endency}),** the self-locking suspension mechanism is achieved, tightly fixing the suture element 200 to the suspension plate 100.

In particular, in order to achieve a friction coefficient which is sufficient to provide a self-locking mechanism, the suture element 200 may be braided from an ultra-high molecular weight polyethylene or co-braided from an ultra-high molecular weight polyethylene and polypropylene, polyester or polyamide, and the front side 100a and/or the rear side 100b of the suspension plate may have a surface roughness of at least 0.6 µm. For example, the suture element 200 may have a diameter of about 0.5 to 0.7 mm, and the first through-hole 11, the second through-hole 12 and/or the third through-hole may have a diameter of about 1.1 mm.

After adjusting the length of the first suspension section 231, the first end section 201 may be shortened, e.g., by cutting, resulting in the configuration shown in Fig. 1G.

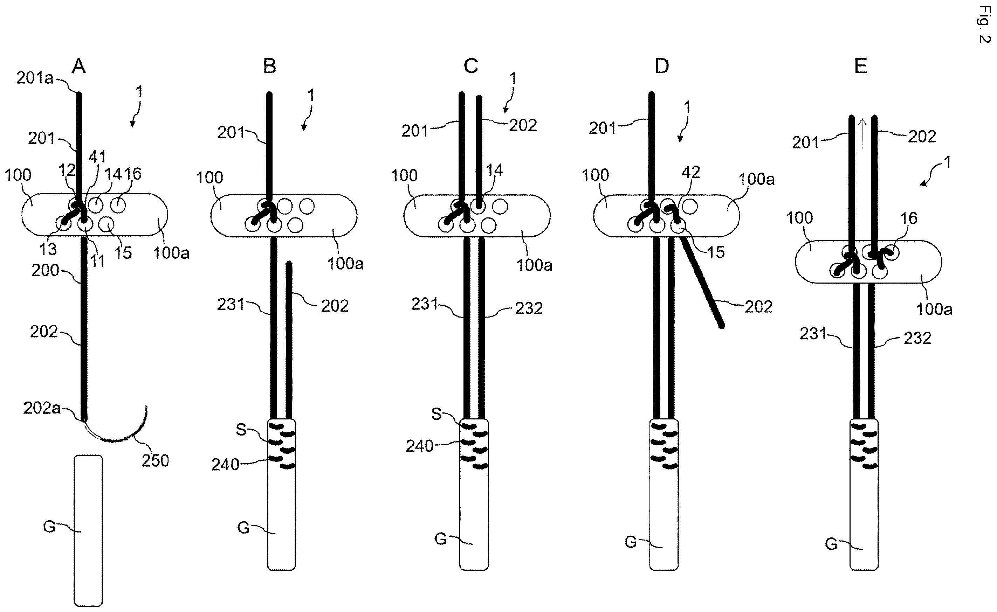

Fig. 2A-E shows the assembly of a further embodiment of an implantable suspension device 1 according to the disclosure comprising a suspension plate 100 with six through-holes 11, 12, 13, 14, 15, 16 and a suture element 200 with a first suspension section 231 and a second suspension section 232. As shown in Fig. 2A, the suture element 200 is particularly engaged with the suspension plate 100 prior to connecting the suture element 200 to the implant G. This pre-assembly is performed as depicted in Fig. 1, wherein the suture element 200 is advanced with the first free end 201a ahead through the first through-hole 11 from the rear side 100b to the front side 100a, through the second through-hole 12 from the front side 100a to the rear side 100b, thereby forming the first locking loop 41, and through the third through-hole 13 from the rear side 100b to the front side 100a and through the first locking loop 41, such that the first end section 201 protrudes from the first locking loop 41 at the front side 100a of the suspension plate 100. Next, a connecting section 240 of the second end section 202 protruding from the rear side 100 B of the suspension plate 100 is connected to the implant G by stitches S. In contrast to the embodiment shown in Fig. 1, the connecting section 240 is not positioned at the second end 202a of the suture element 200. Accordingly, a part of the second end section 202 extends between the connecting section 240 and the second end 202a (Fig. 2B). However, in the same manner as in the embodiment of Fig. 1, the connecting section 240 of the suture element 200 may be stitched to the implant G by means of a needle 250 attached to the second end 202a of the suture element 200 as shown in Fig. 2A. After stitching the connecting section 240 to the implant G, the needle 250 may then be removed by cutting the suture element 200.

To form the assembly shown in Fig. 2E, the second end section 202 is inserted into the fourth through-hole 14 from the rear side 100b to the front side 100a of the suspension plate 100, with the second end 202a ahead (Fig. 2C).

Subsequently, the second end section 202 is advanced through a fifth through-hole 15 from the front side 100a to the rear side 100b of the suspension plate 100 with the second end 202a ahead, thereby forming a second locking loop 42 on the front side 100a of the suspension plate 100 (Fig. 2D).

The second end section 202 is then inserted into a sixth through-hole 16 from the rear side 100b to the front side 100a of the suspension plate 100 with the second end 202a ahead and advanced through the second locking loop 42 on the front side 100a of the suspension plate 100 (Fig. 2E).

Thereby, a first suspension section 231 of the suture element 200 protruding from the rear side 100b of the suspension plate 200 is formed between the first through-hole 11 and the implant G, and a second suspension section 232 of the suture element 200 protruding from the rear side 100b of the suspension plate 200 is formed between the implant G and the fourth through-hole 14.

In the final assembly depicted in Fig. 2E, the first end section 201 of the suture element 200 protrudes from the first locking loop 41 on the front side 100a of the suspension plate 100, and the second end section 202 protrudes from the second locking loop 42 on the front side 100a of the suspension plate 100.

The length of the first and second suspension strand 231, 232 can then be adjusted independently of each other by pulling on the first end section 201 or the second end section 202, respectively (see arrow in Fig. 2E). Of course, the length of both suspension strands 231, 232 can also be adjusted simultaneously.

The self-locking mechanism of the first locking loop 41 and the second locking loop 42 functions in the same way as described above for the embodiment shown in Fig. 1 and the same forces act on each suspension strand 231, 232.

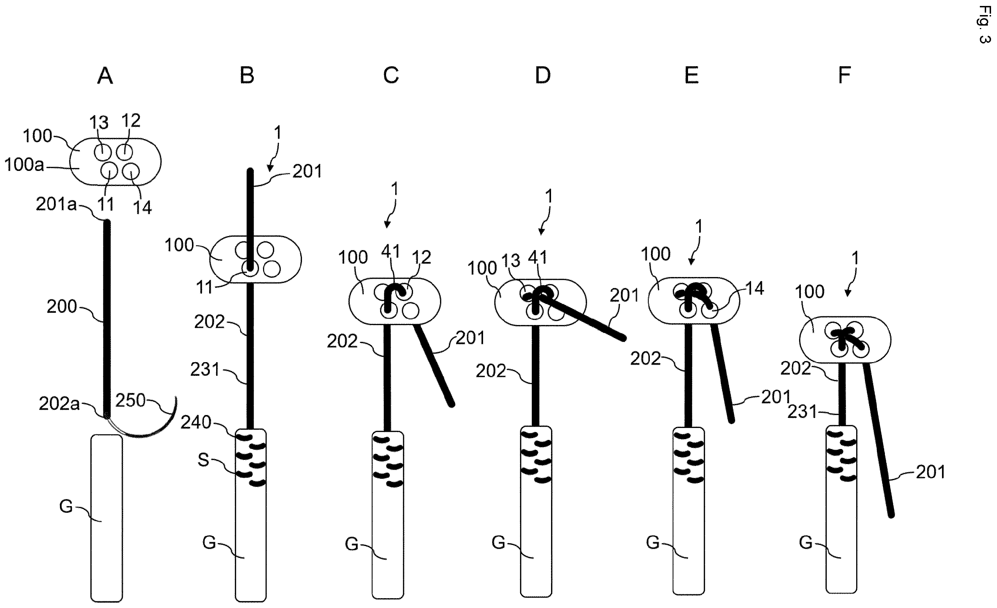

Fig. 3A-F shows the assembly of a further embodiment of the implantable suspension device 1 according to the present disclosure, wherein the suspension plate 100 has four through-holes 11, 12, 13, 14, and the first end section 201 protrudes from the suspension plate 100 on the same side as the first suspension strand 231.

As depicted in Fig. 3A and 3B, the suture element 200 is attached by stitches S to the implant G using a connecting section 240 positioned on the second free end 202a of the suture element 200, similar to the embodiment of Fig. 1. As described above, the connecting section 240 of the suture element 200 may be stitched to the implant G by means of a needle 250 attached to the second end 202a of the suture element 200 as shown in Fig. 3A. After stitching the connecting section 240 to the implant G, the needle 250 may then be removed by cutting the suture element 200. Of course, this step may also be performed after assembling the suspension plate 100 with the suture element 200.

The first free end 201a of the suture element 200 is inserted into the first through-hole 11 of the suspension plate 100 from the rear side 100b to the front side 100a (Fig. 3B), into the second through-hole 12 from the front side 100a to the rear side 100b, thereby forming the first locking loop 41 on the front side 100a (Fig. 3C), into the third through-hole 13 from the rear side 100b to the front side 100a, through the first locking loop 41 (Fig. 3D) and finally into the fourth through-hole 14 from the front side 100a to the back side 100b (Fig. 3E).

Thus, the first suspension section 231 extending between the implant G and the first through-hole 11 and the first end section 201 protruding from the fourth through-hole 14 are both arranged on the rear side 100b of the suspension plate 100, which allows to adjust the length of the first suspension section 231 from the rear side 100b by pulling on the first end section 201. This is advantageous for some applications of the implantable suspension device 1, where the rear side 100b of the suspension plate 100 can be more easily accessed than the front side 100a of the suspension plate 100.

The self-locking mechanism of the first locking loop 41 according to Fig. 3 functions in the same way as described above for the embodiment shown in Fig. 1 and the same forces act on the first suspension strand 231.

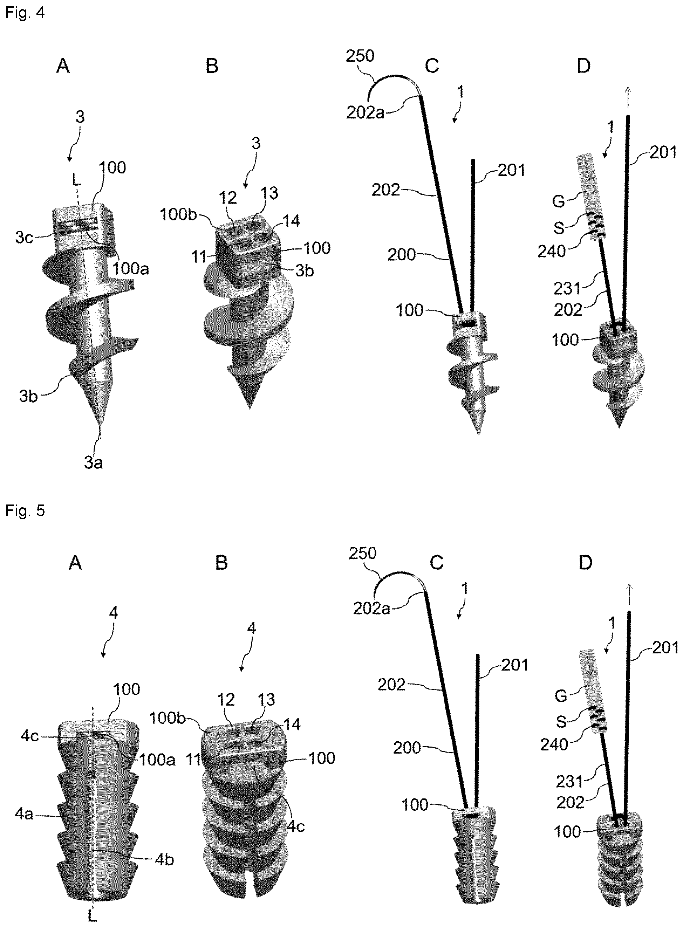

Fig. 4 and 5 show embodiments of the implantable suspension device 1, where the suspension plate 100 is integrally formed with a screw 3 (Fig. 4) or an anchor 4 (Fig. 5) for insertion into a bone.

The screw 3 shown in Fig. 4A extends along a longitudinal axis L between the suspension plate 100 and a pointed tip 3a, wherein the rear side 100b (see Fig. 4B) of the suspension plate 100 forms an end face of the screw 3 which is arranged perpendicular to the longitudinal axis L. The screw 3 further comprises a sharp cutting thread 3b configured to cut into a bone when the screw 3 screwed into the bone with the pointed tip 3a ahead.

The suspension plate 100 integrally formed with the screw 3 comprises a first through-hole 11, a second through-hole 12, a third through-hole 13 and a fourth through-hole 14, each extending between the front side 100a and the rear side 100b of the suspension plate 100. To access the front side 100a of the suspension plate 100, a lateral opening 3c is provided (Fig. 4A-B).

Fig. 5A shows an anchor 4 for insertion into a bore hole in a bone, which extends along a longitudinal axis L, wherein the rear side 100b (see Fig. 5B) of the suspension plate 100 forms an end face of the anchor 4 which is arranged perpendicular to the longitudinal axis L. The anchor 4 comprises a plurality of teeth 4a for engaging the inside walls of the bore hole in the bone to generate friction in order to tightly fix the anchor 4 in the bore hole. Furthermore, the anchor 4 comprises a groove 4b extending along the longitudinal axis L which groove 4b allows the teeth 4a to be pressed together during insertion into the bore hole.

The suspension plate 100 integrally formed with the anchor 4 comprises a first through-hole 11, a second through-hole 12, a third through-hole 13 and a fourth through-hole 14, each extending between the front side 100a and the rear side 100b of the suspension plate 100. To access the front side 100a of the suspension plate 100, a lateral opening 4c is provided (Fig. 5A-B).

As indicated in Fig. 4C and 5C, a suture element 200 can be inserted into the through-holes 11, 12, 13, 14 of the suspension plate 100, particularly in the same way as described for the embodiment of Fig. 3, thereby forming a locking loop for self-locking the suture element 200 on the suspension plate and resulting in a first suspension section 231 between a connecting section 240 attached to an implant G and the suspension plate 100 and a first end section 201 configured to be pulled to adjust the length of the first suspension section 231, wherein both the first suspension section 231 and the first end section 201 protrude from the same side (that is the rear side 100b) of the suspension plate 100 (Fig. 4C-D and 5C-D).

A needle 250 is attached to the second end 202a of the suture element 200 as shown in Fig. 4C and 5C to attach the connecting section 240 (positioned on the second end 202a) to the implant G by stitches S. Of course, the second end 202a of the suture element 200 may also be free, wherein the connecting section 240 is attached to the implant G e.g. by a separate needle. Furthermore, a needle 250 may also be attached to the second end 202a of the suture element 200 of the suspension devices 1 shown in Fig. 1, Fig. 2 or Fig. 3 in the same manner as illustrated in Fig. 4 and Fig. 5.

For example, the implantable suspension device 1 according to Fig. 4 and Fig. 5 may be used in the following manner: the screw 3 or anchor 4 is inserted into a bone with a dedicated screw driver or anchor driver, particularly with the first suspension section 231 and the first end section 201 embedded in a tube of the screw driver or anchor driver. Next, the implant G is stitched together with the connecting section 240 of the suture element 200. Finally, the first end section 201 is pulled to adjust the length of the first suspension section 231.

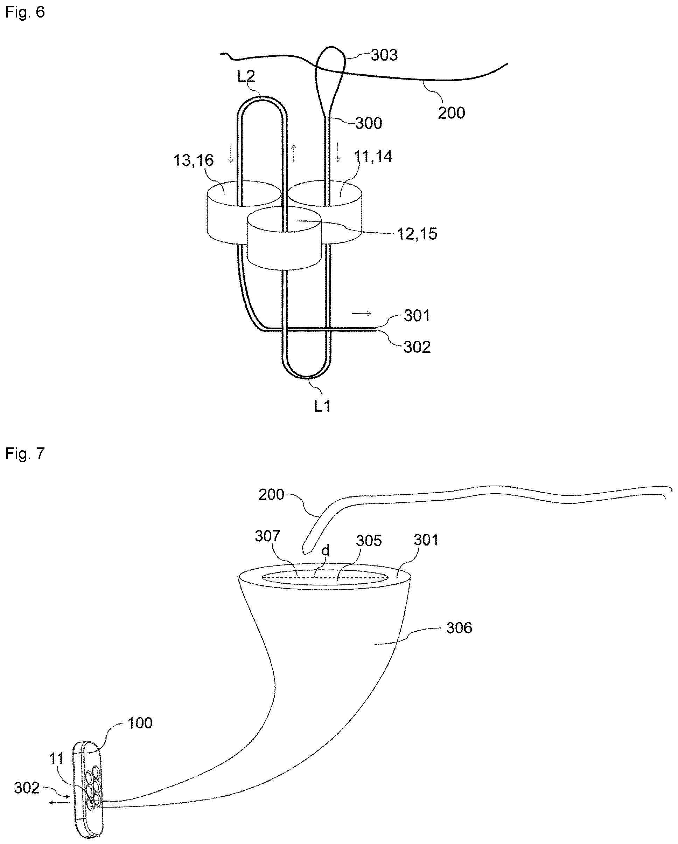

Fig. 6 schematically shows a method of assembling the suture element 200 with the suspension plate 100 to form the implantable suspension device 1 according to the disclosure.

A guiding rope or guiding wire 300 is pre-assembled with the suspension plate 100 by inserting the guiding rope 300 into the through-holes 11, 12, 13, 14, 15, 16 of the suspension plate 100 in the same configuration as later desired for the suture element 200. In Fig. 6, three through-holes are schematically depicted, but of course the principle explained hereafter can also be applied to a suspension plate 100 with four or six through holes (see Fig. 2 and Fig. 3) within the scope of the disclosure.

The guiding rope 300 extends from a first end 301 successively through the third through-hole 13 or sixth through-hole 16, the second through-hole 12 or fifth through-hole 15, and the first through-hole 11 or fourth through-hole 14, forms a loop 303 and extends back through the first through-hole 11 or fourth through-hole 14, the second through-hole 12 or fifth through-hole 15 and the third through-hole 13 or the sixth through-hole 16 towards a second end 302. An additional first loop L1 or bend is formed between the first or fourth through-hole 11, 14 and the second or fifth through-hole 12, 15 and a further second loop L2 or bend is formed between the second or fifth through-hole 12, 15 and the third or sixth through-hole 13. 16.

When the suture element 200 is inserted into the loop 303 of the guiding rope 300 as shown in Fig. 6, the loop 303 may be successively advanced along the arrows depicted in Fig. 6 through the first or fourth through-hole 11, 14, the second or fifth through-hole 12, 15 and the third or sixth through-hole 13, 16 along with the suture element 200, resulting in the engagement of the suture element 200 with the suspension plate 100 shown in Fig. 1-3.

Fig. 7 depicts an alternative embodiment of the guiding rope 300, which comprises an inner volume 305 extending between a first end 301 and a second end 302 of the guiding rope 300. The guiding rope 300 is configured to receive the suture element 200 in the inner volume 305 and the guiding rope 300 forms a funnel 306 at the first end 301, the funnel 306 defining an opening 307 to receive the suture element 200 in the inner volume 305. In Fig. 7, an expanded state of the funnel 306 is shown, where the opening 207 has a diameter d. The funnel 306 is further characterized by a collapsed state, wherein the opening 307 has a diameter d which is smaller than in the depicted expanded state.

The guiding rope 300 according to Fig. 7 can be pre-assembled with the suspension plate 100 in the same manner as shown in Fig. 6, wherein the guiding rope 300 is inserted into the through-holes 11, 12, 13, 14, 15, 16 of the suspension plate 100 in the collapsed state. Then, the suture element 200 can be inserted into the inner volume 305 using the expanded state of the funnel 306 to easily insert the suture element 200 into the inner volume 305. To assemble the implantable suspension device 1, the guiding rope 300 can be moved together with the suture element 200 disposed in the inner volume 305 resulting in the suture element 200 engaging with the suspension plate 100 as explained above in relation to Fig. 6.

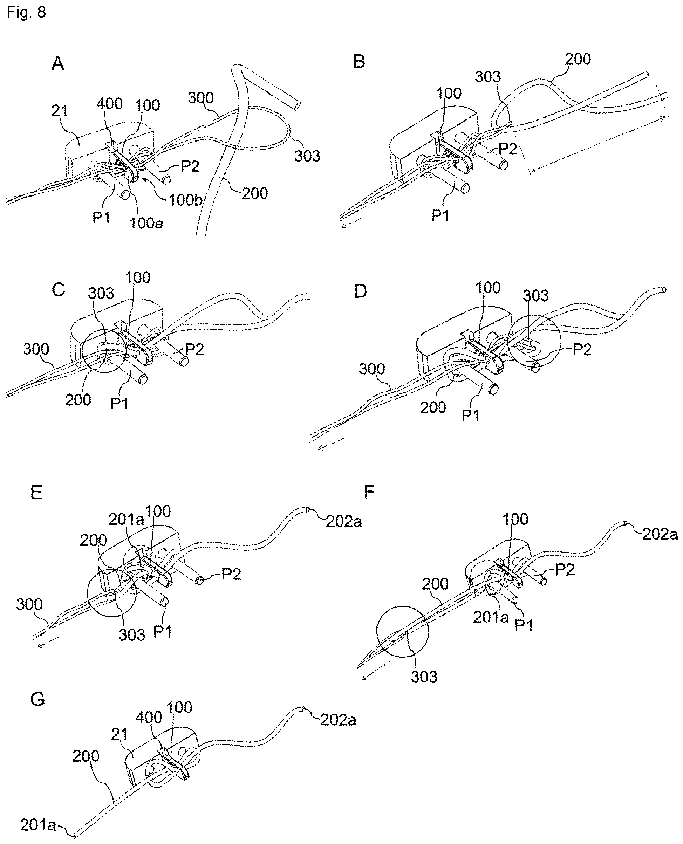

Hereafter, an assembly device 2 for assembling the implantable suspension device 1 according to the present disclosure is described with reference to Figures 8-13.

Fig. 8A-G show a holding part 21 of the assembly device 2 holding a suspension plate 100 with a pre-arranged guiding rope 300 as well as steps of assembling the suspension plate 100 with the suture element 200 using the assembly device 2 to form the implantable suspension device 1.

The holding part 21 comprises a holder 400 formed as a slot for insertion of the suspension plate 100, a first pin P1 and a second pin P2, wherein the first pin P1 and the second pin P2 are arranged on opposite sides of the holder 400. The holder 400 is arranged between the pins P1, P2 such that the first pin P1 faces the front side 100a of the suspension plate 100 and the second pin P2 faces the rear side 100b of the suspension plate 100. In Fig. 8, a suspension plate 100 with six through-holes 11, 12, 13, 14, 15, 16 is shown (see Fig. 2), but of course the assembly device 2 described here is also suitable for suspension plates with three (Fig. 1) or four (Fig. 3) through-holes 11, 12, 13, 14.

As shown in Fig. 8A, a guiding rope 300 extends through the first, the second and the third through-hole 11, 12, 13 in the same manner as schematically depicted in Fig. 6, and forms a loop 303 enlacing the suture element 200. Therein, the first loop L1 of the guiding wire 300 indicated in Fig. 6 is laid around the first pin P1 and the second loop L2 of the guiding wire 300 is laid around the second pin P2 to avoid entanglement of the guiding wire 300 and suture element 200 and avoid possible blocking of the assembly mechanism.

Fig. 8B-G show how the loop 303 of the guiding rope 300 is advanced through the through-holes of the suspension plate 100 along with the suture element 200 to pull the suture element 200 through the through-holes (in the same manner as schematically illustrated in Fig. 6) winding around the first pin P1 and the second pin P2, and resulting in engagement of the suture element 200 with the suspension plate 100 as shown in Fig. 1-3.

In Fig. 8C-F the loop 303 of the guiding rope 300 is marked with a solid circle and in Fig. 8E-F the first free end 201a of the suture element is marked with a dashed circle. As can be seen in Fig. 8F and 8G, after having passed through the first, the second and the third through-hole 11, 12, 13, the first end 201a of the suture element 200 slips through the loop 303 of the guiding rope 300 and detaches while being pulled.

Fig. 8G shows the final configuration of the suture element 200 in the suspension plate 100 held by the holder 400 of the holding part 21 after the guiding rope 300 has been removed. This setup is the same as the one shown in Fig. 2.

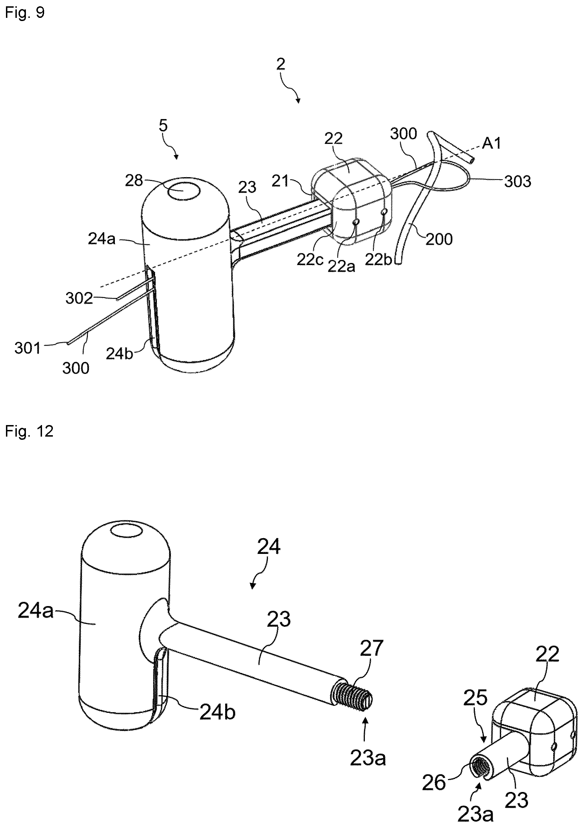

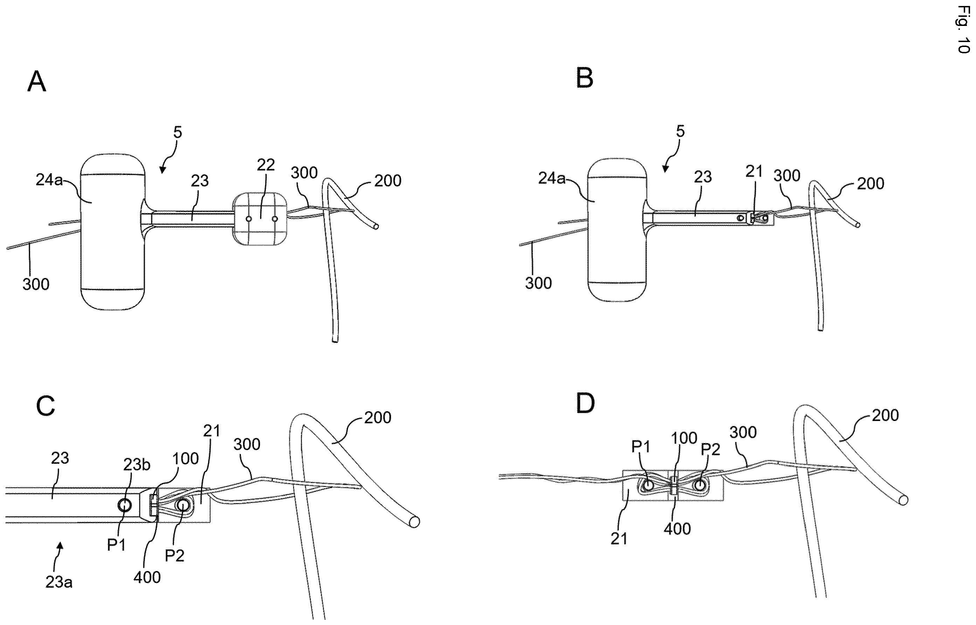

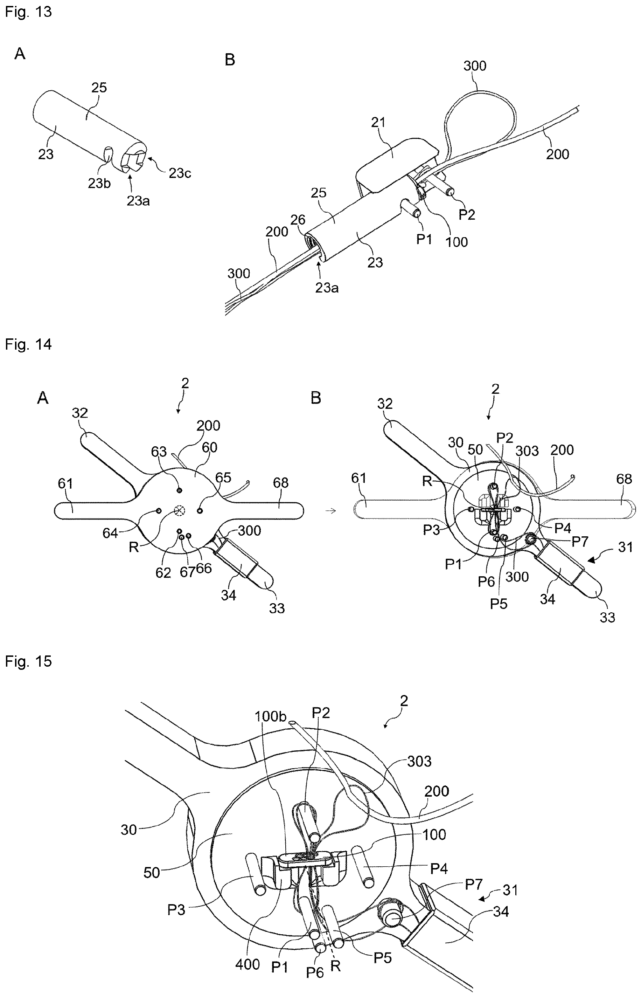

Fig. 9 shows an assembly device 2 comprising a holding part 21 (same as depicted in Fig. 8) assembled with a casing part 22 and a tool 5 for implanting the implantable suspension device 1 at a desired location in a human or animal body. The tool 5 comprises a guide bar 23 for guiding the suture element 200 and/or the guiding rope 300, extending along a first axis A1 and connected to the holding part 21 and the casing part 22, and a handle 24a for holding the tool 5, wherein the handle 24a is connected to the guide bar 23 at the opposite end in respect of the holding part 21 and the casing part 22. A guiding rope 300 forming a loop 303 that enlaces the suture element 200 extends along the first axis A1 through the casing part 22, along a slot 23a of the guide bar 23 (see Fig. 10C and 13A) and through a groove 24b of the handle 24a, the first end 301 and the second end 302 of the guiding rope 300 protruding from the groove 24b.

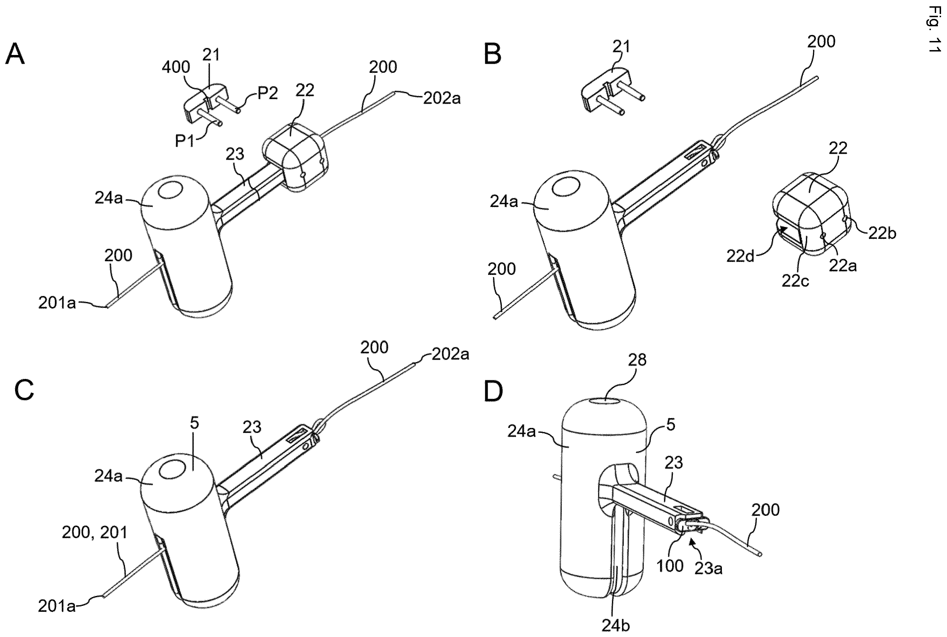

The casing part 22 comprises a first hole 22a and a second hole 22b for receiving the first pin P1 and the second pin P2 of the holding part 21, respectively, and connect the casing part 22 to the holding part 21, and a wall 22c encasing the holding part 21 and comprising an opening 22d for receiving the guide bar 23 (see also Fig. 11B for a detailed view).

Fig. 10A-D illustrate different components of the assembly device 2 shown in Fig. 9. Fig. 10B-D show the assembly device 2 without the casing part 22. As best seen in Fig. 10C, the guiding bar 23 comprises a hole or recess 23b for receiving the first pin P1 of the holding part 21 to connect the guiding bar 23 to the holding part 21. In addition, the guiding bar 23 may comprise a notch 23c positioned at the tip of the guiding bar 23 (see Fig. 13A) for inserting the suspension plate 100. Fig. 10D shows a side view of the holding part 21 of the assembly device 2 with the suspension plate 100 in the holder 400 and the guiding rope 300 extending through the through-holes 11, 12, 13, 14, 15, 16 of the suspension plate 100 and around the pins P1, P2 (see also Fig. 8A).

By pulling the guiding rope 300 through the assembly device 2 along the slot 23a of the guide bar 23 and through the groove 24b of the handle 24a, the suture element 200 may be assembled and engaged with the suspension plate 100 held by the holding part 21, resulting in the first end 201a of the suture element 200 protruding from the groove 24b of the handle 24a and the second end 202a of the suture element 200 protruding from the opening 22d of the casing part 22 opposite the tip of the guide bar 23, resulting in the setup shown in Fig. 11A when the holding part 21 is removed.

As depicted in Fig. 11B-D, the casing part 22 can then be removed from the tip of the guiding bar 23, leaving the suspension plate 100 with the suture element 200 held in the notch 23c at the tip of the guide bar 23.

The second end 202a of the suture element 200 may then be attached to an implant G (see Fig. 1-3) and the tool 5 may subsequently be used to place the suspension plate 100 at the desired location in the human or animal body using the handle 24a to grip the tool 5 and/or apply force to the suspension plate 100 if necessary. By pulling on the first end section 201 of the suture element 200 protruding from the groove 24b of the handle 24a, the length of the suspension section 231 can be adjusted as described above.

The tool 5 may further comprise an actuating element 28 that can be actuated to clamp the suture element 200 in the slot 23a of the guide bar 23 or release a clamping mechanism.

Fig. 12 and 13 show an embodiment of the assembly device 2 where the tool 5 is separable into a handle part 24 comprising the handle 24a and a first portion of the guiding bar 23 and a tip part 25 comprising a second portion of the guiding bar 23 which comprises the recess or hole 23b for receiving the first pin P1 of the holding part 21 and the notch 23c for inserting the suspension plate (see Fig. 13A-B).

As shown in Fig. 12, the tip part 25 comprises an inner thread 26 which corresponds to an outer thread 27 at the tip of the handle part 24, such that the outer thread 27 may be screwed into the inner thread 26 to assemble the handle part 24 and the tip part 25. In particular, the tip part 25 may be preassembled with the holding part 21, casing part 22, suspension plate 100 and guiding rope 300 as indicated in Fig. 12B and 13B, more particularly forming a disposable unit, whereas the handle part 24 of the tool 5 is reusable.

Fig. 14 and 15 illustrate a further embodiment of the assembly device 2 according to the present disclosure. The assembly device 2 comprises a first part 30 and a second part 50 which are rotatable in respect of each other about a rotation axis R (see Fig. 14A).

As depicted in Fig. 15, the second part 50 is formed as a circular plate arranged in a circular recess of the first part 30, such that the second part 50 can be rotated with respect to the first part 30 about the rotation axis R (which extends perpendicular to the circular plate at the center of the circular plate). The second part 50 comprises a holder 400 for holding the suspension plate 100, a first pin P1 and a second pin P2 arranged on opposite sides of the holder 400, such that the first pin P1 faces the front side 100a of the suspension plate 100 and the second pin P2 faces the rear side 100b of the suspension plate 100. The second part 50 further comprises a third pin P3 and a fourth pin P4 arranged at opposite sides of the holder 400 between the first pin P1 and the second pin P2, such that the first, second, third and fourth pin P1, P2, P3, P4 form the corners of an imaginary square along a periphery around the holder 400. A fifth pin P5 and a sixth pin P6 are arranged on the second part 50 adjacent the first pin P1 radially outside of the first pin P1.

The first part 30 comprises a fixing element 31 for fixing the guiding rope 300 to the first part 30. In the embodiment shown in Fig. 14 and 15, the fixing element 31 comprises a brace 34 for bracing or clamping the guiding rope 300 to the first part 30. A seventh pin P7 of the first part 30 is used to wind the guiding rope 300 around the seventh pin P7 and guide the guiding rope 300 to the fixing element 31.

As shown in Fig. 14A, the assembly device further comprises a lid part 60 comprising a first handle 61, a further handle 68 opposite the first handle 61, a first hole 62, a second hole 63 and further holes 64, 65, 66, 67 for receiving the first, second, third, fourth, fifth and sixth pin P1, P2, P3, P4, P5, P6 of the second part 50 (see Fig. 14B and 15) to couple the lid part 60 to the second part 50.

The first part 30 further comprises a second handle 32 and a further handle 33 opposite the second handle 32.

Fig. 14B and 15 further show a guiding rope 300 inserted into the through-holes 11, 12, 13, 14, 15, 16 of the suspension plate 100 as schematically depicted in Fig. 6 (with the first loop L1 around the first pin P1 and the second loop L2 around the second pin P2. The guiding rope 300 forms a loop 303 that enlaces a suture element 200, extends through three of the through-holes 11, 12, 13 of the suspension plate 100 in the holder 400 and extends between the fifth pin P5 and the sixth pin P6 towards the fixing element 31 on the first part 30.

When the lid part 60 is coupled to the second part 50 and the first handle 61 of the lid part 60 is moved relative to the second handle 32 of the first part 30, the second part 50 is rotated about the rotation axis R with respect to the first part 30. Thereby, the fixing element 31with the ends of the guiding rope 300 attached revolves around the second part 50 and the loop 303 of the guiding rope 300 is drawn around the pins P1, P2 and through the through-holes 11, 12, 13 of the suspension plate 100 in the holder 400, resulting in an engagement of the suture element 200 with the suspension plate 100 similar to the procedure shown in Fig. 8A-G.

Due to the rotary motion of the guiding rope 300, the assembly advantageously requires less space than in case of a linear motion. In addition, uncontrollable movements during the mechanism due to different forces acting during different stages of assembly, are avoided by the rotary assembly procedure. Furthermore, by using sufficiently long handles, the force applied to the handles can be amplified (e.g. by a factor of about 3), resulting in less force applied by the user. The pins P1, P2, P3, P4 ensure that the guiding rope 300 and the suture element 200 do not entangle and the pins P5, P6, P7 serve to guide the guiding rope towards the fixing element 31.

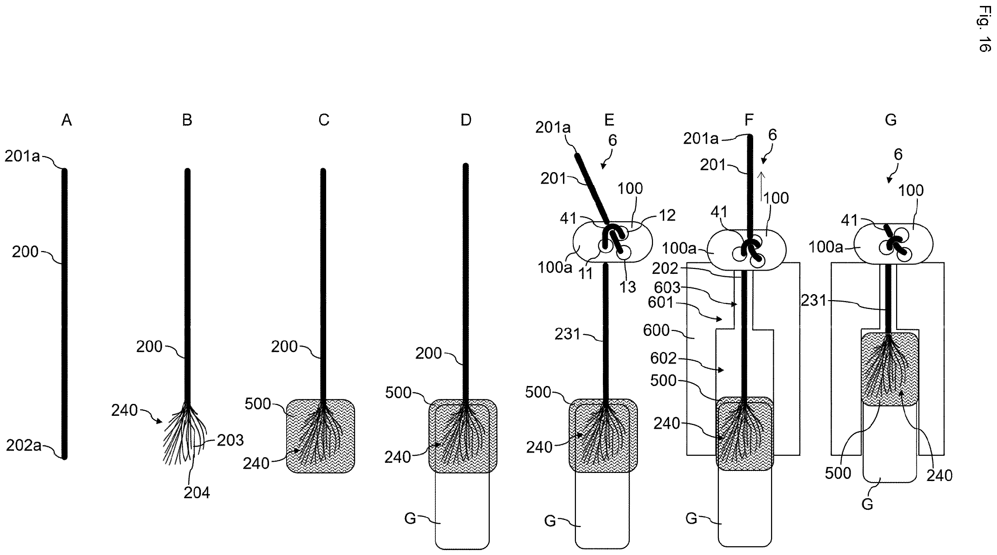

Fig. 16 A-G show assembly steps of a medical implant 6 according to an embodiment of the disclosure comprising a medical textile 500, e.g. from a felt material, and an implantable suspension device 1 according to the disclosure which comprises a suspension plate 100 and a suture element 200 comprising a first end 201a and a second end 202a (Fig. 16A).

As shown in Fig. 16B, the suture element 200 comprises at the second end 202a, a connecting section 240 composed of separated fibers 203, each comprising a fiber end 204. The fibers 203 are spread out in a fanlike manner, thereby increasing the area of the connecting section 240. In particular, the entire suture element 200 is formed from braided or twisted fibers 203, which have been separated from each other at the second end 202a to form the connecting section 240.

Next, as illustrated in Fig. 16C, the connecting section 240 of the suture element 200 is connected to a medical textile 500 in form of a patch. In particular, to connect the medical textile 500 to the connecting section 240, the medical textile 500 and the connecting section 240 are placed on top of each other and a felting needle comprising at least one barb is repeatedly advanced through the connecting section 240 and the medical textile 500. Thereby, the fibers 203 of the connecting section 240 are interwoven with the material of the medical textile 502, resulting in a strong connection. In case the medical textile 500 is formed from a felt material, advancing the felting needle through the connecting section 240 and the medical textile 500 also results in pulling out fibers from the felt material of the medical textile 500, which further contributes to the connection.

Subsequently, the medical textile 500, which is now attached to the suture element 200, is connected to a flexible implant G, e.g., a soft tissue such as a tendon (Fig. 16D). This is particularly achieved by placing the medical textile 500 onto the flexible implant G and repeatedly advancing a felting needle having at least one barb through the medical textile 500 into the flexible implant G, which results in pulling out fibers from the material, e.g., the felt material, of the medical textile 500 (and particularly also fibers 203 of the connecting section 240 of the medical suture 200) and inserting these fibers into the flexible implant G. Thereby, a strong connection between the flexible implant G and the medical textile 500, as well as the attached suture element 200 can be obtained in a relatively easy manner.

Fig. 16 E illustrates the assembly of the suture element 200 with the suspension plate 100 to form the medical implant 6, which includes an implantable suspension device 1 composed of the suture element 200 and the suspension plate 100. According to this example, the suspension plate 100 comprises three through holes 11, 12, 13, which are arranged as in the example of the implantable suspension device 1 shown in Fig. 1. The first end 201a of the suture element 200 is advanced through the through holes 11, 12, 13, and a first locking loop 41 is formed as described above and illustrated in Fig. 1. Furthermore, as described above, the section of the suture element 200 between the suspension plate 100 and the connecting element 200/medical textile 500 forms a first suspension section 231, the length of which can be adjusted by pulling on the first end section 201.

Finally, Fig. 16F and 16G show the insertion of the medical implant 6 into a bone tunnel 601 of a bone 600 to fix the flexible implant G on the bone 600, e.g. during anterior cruciate ligament (ACL) repair. The bone tunnel 601 comprises a first segment 602 and an adjacent second segment 603 with a smaller diameter compared to the first segment 602. The diameter of the first segment 602 particularly matches the approximate width of the flexible implant G, and the second segment 603 has dimensions allowing the suspension plate 100 to be moved through the second segment 603.

During a typical surgical procedure, the bone tunnel 601 is first introduced into the bone 600 using a surgical drill. Subsequently, the suspension plate 100 with the attached suture element 200 is introduced into the first segment 602 of the bone tunnel 601 and then moved through the second segment 603 of the bone tunnel 601 to the outside of the bone 600. The suspension plate 100 is then flipped to be arranged perpendicular to the bone tunnel 601. Next, the first suspension section 231 is shortened by pulling on the first end section 201, which results in the medical textile 500 with the attached flexible implant G being pulled into the first segment 602 of the bone tunnel 601. After tightening the first suspension section 231, the first end section 201 of this is suture element 200 may be cut, resulting in the configuration shown in Fig. 16 G.

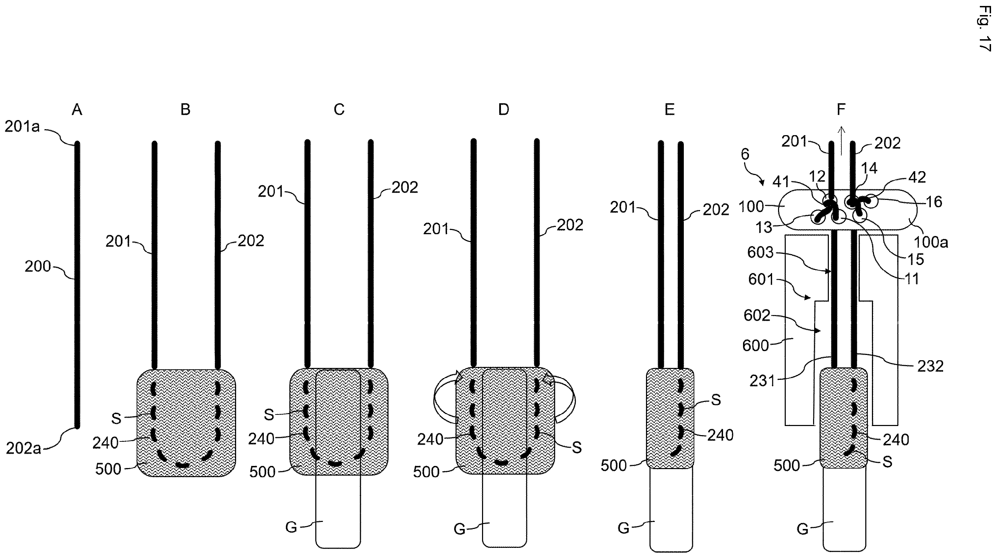

Fig. 17 A-F show assembly steps of a medical implant 6 according to a further embodiment of the disclosure comprising a medical textile 500, e.g. from a felt material, and an implantable suspension device 1 according to the disclosure which comprises a suspension plate 100 and a suture element 200 comprising a first end 201a and a second end 202a (Fig. 17A).

As shown in Fig. 17 B, a central connecting section 240 of the suture element 200 is connected to a medical textile 500, e.g. a patch from a felt material, by stitches S, resulting in a first end section 201 and a second end section 202 protruding from the medical textile 500. The stitch S connection of the suture element 200 and the medical textile 500 results in an improved tensile strength of the medical textile 500.

Fig. 17 C to Fig. 17 E illustrate how a flexible implant G is connected to the medical textile 500 according to an example of the disclosure. As shown in Fig. 17 C, the medical textile 500 with the attached suture element 200 is first placed on the flexible implant G. Next, the medical textile 500 is at least partially wrapped around the flexible implant G as indicated by the arrows in Fig. 17 D, thereby forming a tubular structure which is arranged around a part of the flexible implant G (Fig. 17 E). Subsequently, the medical textile 500 is connected to the flexible implant G, in particular, by repeatedly advancing a felting needle comprising at least one barb through the medical textile 500 and the flexible implant G, which results in fibers of the medical textile 500 being inserted into the flexible implant G. Optionally, the medical textile 500 may additionally be pre-attached to the flexible implant G, e.g. by repeatedly advancing a felting needle through the assembly shown in Fig. 17 C.

Finally, as illustrated in fig. 17 F, a suspension plate 100 having six through holes 11, 12, 13, 14, 15, 16 is assembled with the first end section 201 and the second end section 202 of the suture element 200, and a first locking loop 41 and a second locking loop 42 are formed in a similar manner as described above and shown in Fig. 2.

The assembled medical implant 6 is then inserted into a bone tunnel 601 as described above for the example shown in Fig. 16, and the first suspension section 231 and second suspension section 232 formed between the suspension plate 100 and the medical textile 500 /connecting section 240 are shortened by pulling on the first end section 201 and the second end section 202 (Fig. 17G).

**List of reference numerals**

| | |
|---|---|
| 1 | Implantable suspension device |
| 2 | Assembly device |
| 3 | Screw |
| 3a | Tip |
| 3b | Cutting thread |
| 3c | Lateral opening |
| 4 | Anchor |
| 4a | Tooth |
| 4b | Groove |
| 4c | Lateral opening |
| 5 | Tool |
| 6 | Medical implant |
| 11 | First through-hole |
| 12 | Second through-hole |
| 13 | Third through-hole |
| 14 | Fourth through-hole |
| 15 | Fifth through-hole |
| 16 | Sixth through-hole |
| 21 | Holding part |
| 22 | Casing part |
| 22a | First hole |
| 22b | Second hole |
| 22c | wall |
| 22d | opening |
| 23 | Guide bar |
| 23a | Slot |
| 23b | hole |
| 23c | Notch |
| 23d | Tip |
| 24 | Handle part |
| 24a | Handle |
| 24b | Groove |
| 25 | Tip part |
| 26 | Inner thread |
| 27 | Outer thread |
| 28 | Actuating element |
| 30 | First part |
| 31 | Fixing element |
| 32 | Second handle |
| 33 | Further handle |
| 34 | Brace |
| 41 | First locking loop |
| 42 | Second locking loop |
| 50 | Second part |
| 60 | Lid part |
| 61 | First handle |
| 62 | First hole |
| 63 | Second hole |
| 64, 65, 66, 67 | Further hole |
| 68 | Further handle |
| 100 | Suspension plate |
| 100a | Front side |
| 100b | Rear side |
| 200 | Suture element |
| 201 | First end section |
| 201a | First end |
| 202 | Second end section |
| 202a | Second end |
| 203 | Fiber |
| 204 | Fiber end |
| 231 | First suspension section |
| 232 | Second suspension section |
| 240 | Connecting section |
| 250 | Needle |
| 300 | Guide rope |
| 301 | First end of the guiding rope |
| 302 | Second end of the guiding rope |
| 303 | Loop |
| 305 | Inner volume |
| 306 | Funnel |
| 307 | Opening of the funnel |
| 400 | Holder |
| 500 | Medical textile |
| 600 | Bone |
| 601 | Bone tunnel |
| 602 | First segment |
| 603 | Second segment |
| P1 | First pin |
| P2 | Second pin |
| P3 | Third pin |
| P4 | Fourth pin |
| P5 | Fifth pin |
| P6 | Sixth pin |
| P7 | Seventh pin |
| d | diameter |
| L | Longitudinal axis |
| L1 | First loop |
| L2 | Second loop |
| R | Rotation axis |
| A1 | First axis |
| F_{L} | Locking force |
| G | Implant |
| S | Stitches |

## Claims

1. An implantable suspension device (1) for fixing an elongated flexible implant (G) in a desired position, comprising: a suspension plate (100) and a suture element (200) engaging with the suspension plate (100), wherein the suture element (200) comprises a first end section (201) extending from said suspension plate (100) to a first end (201a) of said suture element (200) and a second end section (202) extending from said suspension plate (100) to a second end (202a) of said suture element (200), wherein the second end section (202) comprises a connecting section (240) configured to be connected to said implant (G) or to a medical textile (500), wherein said suture element (200) comprises or consists of a plurality of fibers (203), wherein the fibers (203) are separated from each other in the connecting section (240), **characterized in that** said connecting section (240) forms a fan-like structure extending in a plane parallel to an extension direction of the suture element (200).

2. The implantable suspension device (1) according to claim 1 **characterized in that** said connecting section (240) is positioned at the second end (202a) of the suture element (200).

3. The implantable suspension device (1) according to claim 1 or 2, **characterized in that** the suture element (200) comprises a needle (250) attached to the suture element (200), particularly to the first end (201a) or the second end (202a).

4. The implantable suspension device (1) according to one of the preceding claims, **characterized in that** the suture element (200) forms a first locking loop (41) for pressing the first end section (201) of the suture element (200) against the suspension plate (100) and thereby locking the first end section (201) with respect to the suspension plate (100).

5. The implantable suspension device (1) according to one of the preceding claims, **characterized in that** the second end section (202) comprises an adjustable first suspension section (231) between said suspension plate (100) and said connecting section (240), wherein the first suspension section (231) is configured to be shortened by pulling on the first end section (201) of the suture element (200), wherein particularly said first end section (201) protrudes out of said first locking loop (41).

6. The implantable suspension device (1) according to one of the preceding claims, **characterized in that** the suspension plate (100) comprises a front side (100a) and a rear side (100b), which rear side (100b) faces away from the front side (100a).

7. The implantable suspension device (1) according to claim 6, **characterized in that** the suspension plate (100) comprises a plurality of through-holes (11, 12, 13, 14, 15, 16), each through-hole (11, 12, 13, 14, 15, 16) extending from the front side (100a) to the rear side (100b) of said suspension plate (100), wherein said suture element (200) extends through said through-holes (11, 12, 13, 14, 15, 16).

8. The implantable suspension device (1) according to claim 7, **characterized in that** said plurality of through-holes (11, 12, 13, 14, 15, 16) is formed by at least three through-holes (11, 12, 13), particularly 3, 4, or 6 through-holes.

9. The implantable suspension device (1) according to claim 7 or 8, **characterized in that** the suture element (200) extends through a first-through-hole (11) of said plurality of through-holes (11, 12, 13, 14, 15, 16) from the rear side (100b) to the front side (100a) of the suspension plate (100), with the first end (201a) ahead, particularly such that the adjustable first suspension section (231) is formed on the rear side (100b) of the suspension plate (100), and wherein preferably the suture element (200) extends through a second through-hole (12) of said plurality of through-holes from the front side (100a) to the rear side (100b) of the suspension plate (100), with the first end (201a) ahead, particularly such that the first locking loop (41) is formed on the front side (100a) of the suspension plate (100), wherein the suture element (200) further extends through a third through-hole (13) of said plurality of through-holes (11, 12, 13, 14, 15, 16) from the rear side (100b) to the front side (100a) of the suspension plate (100) with the first end (201a) ahead, wherein particularly the first end section (201) of the suture element (200) further extends through the first locking loop (41) for clamping the first end section (201) to the front side (100a) of the suspension plate (100) by means of the first locking loop (41) with a locking force (F_{L}), and wherein preferably the suture element (200) further extends through a fourth through-hole (14) of said plurality of through-holes from the front side (100a) to the rear side (100b) of the suspension plate (100) with the first end (201a) ahead, and wherein preferably the suture element (200) further extends through a fourth through-hole (14) of said plurality of through-holes (11, 12, 13, 14, 15, 16) from the rear side (100b) to the front side (100a) of the suspension plate (100) with the second end (202a) ahead, particularly such that the suture element (200) forms a second suspension section (232) between said connecting section (240) and said suspension plate (100), wherein said suture element (200) further extends through a fifth through-hole (15) of said plurality of through-holes (11, 12, 13, 14, 15, 16) from the front side (100a) to the rear side (100b) of the suspension plate (100) with the second end (202a) ahead, particularly such that a second locking loop (42) is formed, wherein the suture element (200) further extends through a sixth through-hole (16) of said plurality of through-holes (11, 12, 13, 14, 15, 16) from the rear side (100b) to the front side (100a) of the suspension plate (100) with the second end (202a) ahead, wherein particularly the second end section (202) of the suture element (200) further extends through said second locking loop (42) for clamping the second end section (202) of the suture element (200) to the front side (100a) of the suspension plate (100) by means of the second locking loop (42) with a locking force (F_{L}), and wherein preferably the suture element (200) comprises an adjustable second suspension section (232) between said connecting section (240) and said suspension plate (100), wherein the second suspension section (232) is configured to be shortened by pulling on the second end section (202) of the suture element (200), wherein particularly said second end section (202) protrudes out of said second locking loop (42).

10. A medical implant (6) comprising the implantable suspension device (1) according to one of the claims 1 to 9 and a medical textile (500), particularly comprising or consisting of a felt material, wherein the connecting section (240) of the suture element (200) is connected to the medical textile (500), wherein the medical textile (500) is configured to be connected to said implant (G)."

11. The medical implant (6) according to claim 10, **characterized in that** the connecting section (240) is connected to the medical textile (500) by stitches (S).

12. The medical implant (6) according to one of the claims 10 to 11, **characterized in that** the medical textile (500) comprises a tubular shape, wherein the medical textile (500) comprises an inner surface configured to be connected to said implant (G).

## Patentansprüche

1. Implantierbare Aufhängevorrichtung (1) zum Fixieren eines länglichen flexiblen Implantats (G) in einer gewünschten Position, umfassend: eine Aufhängungsplatte (100) und ein Nahtelement (200), das mit der Aufhängungsplatte (100) in Eingriff steht, wobei das Nahtelement (200) einen ersten Endabschnitt (201) umfasst, der sich von der Aufhängungsplatte (100) zu einem ersten Ende (201a) des Nahtelements (200) erstreckt, und einen zweiten Endabschnitt (202), der sich von der Aufhängungsplatte (100) zu einem zweiten Ende (202a) des Nahtelements (200) erstreckt, wobei der zweite Endabschnitt (202) einen Verbindungsabschnitt (240) umfasst, der dazu ausgebildet ist, mit dem Implantat (G) oder mit einem medizinischen Textil (500) verbunden zu werden, wobei das Nahtelement (200) eine Mehrzahl von Fasern (203) umfasst oder aus einer Mehrzahl von Fasern (203) besteht, wobei die Fasern (203) im Verbindungsabschnitt (240) voneinander beabstandet sind, **dadurch gekennzeichnet, dass** der Verbindungsabschnitt (240) eine fächerartige Struktur bildet, die sich in einer Ebene parallel zu einer Erstreckungsrichtung des Nahtelements (200) erstreckt.

2. Implantierbare Aufhängevorrichtung (1) nach Anspruch 1, **dadurch gekennzeichnet, dass** der Verbindungsabschnitt (240) am zweiten Ende (202a) des Nahtelements (200) angeordnet ist.

3. Implantierbare Aufhängevorrichtung (1) nach Anspruch 1 oder 2, **dadurch gekennzeichnet, dass** das Nahtelement (200) eine Nadel (250) umfasst, die am Nahtelement (200) befestigt ist, insbesondere am ersten Ende (201a) oder am zweiten Ende (202a).

4. Implantierbare Aufhängevorrichtung (1) nach einem der vorhergehenden Ansprüche, **dadurch gekennzeichnet, dass** das Nahtelement (200) eine erste Verriegelungsschlaufe (41) bildet, um den ersten Endabschnitt (201) des Nahtelements (200) gegen die Aufhängungsplatte (100) zu drücken und dadurch den ersten Endabschnitt (201) relativ zur Aufhängungsplatte (100) zu verriegeln.

5. Implantierbare Aufhängevorrichtung (1) nach einem der vorhergehenden Ansprüche, **dadurch gekennzeichnet, dass** der zweite Endabschnitt (202) einen einstellbaren ersten Aufhängungsabschnitt (231) zwischen der Aufhängungsplatte (100) und dem Verbindungsabschnitt (240) umfasst, wobei der erste Aufhängungsabschnitt (231) dazu ausgebildet ist, durch Ziehen am ersten Endabschnitt (201) des Nahtelements (200) verkürzt zu werden, wobei insbesondere der erste Endabschnitt (201) aus der ersten Verriegelungsschlaufe (41) herausragt.

6. Implantierbare Aufhängevorrichtung (1) nach einem der vorhergehenden Ansprüche, **dadurch gekennzeichnet, dass** die Aufhängungsplatte (100) eine Vorderseite (100a) und eine Rückseite (100b) umfasst, wobei die Rückseite (100b) von der Vorderseite (100a) weg weist.

7. Implantierbare Aufhängevorrichtung (1) nach Anspruch 6, **dadurch gekennzeichnet, dass** die Aufhängungsplatte (100) eine Mehrzahl von Durchgangsöffnungen (11, 12, 13, 14, 15, 16) umfasst, wobei jede Durchgangsöffnung (11, 12, 13, 14, 15, 16) sich von der Vorderseite (100a) zur Rückseite (100b) der Aufhängungsplatte (100) erstreckt, wobei sich das Nahtelement (200) durch diese Durchgangsöffnungen (11, 12, 13, 14, 15, 16) erstreckt.

8. Implantierbare Aufhängevorrichtung (1) nach Anspruch 7, **dadurch gekennzeichnet, dass** die Mehrzahl von Durchgangsöffnungen (11, 12, 13, 14, 15, 16) durch mindestens drei Durchgangsöffnungen (11, 12, 13), insbesondere 3, 4 oder 6 Durchgangsöffnungen, gebildet ist.

9. Implantierbare Aufhängevorrichtung (1) nach Anspruch 7 oder 8, **dadurch gekennzeichnet, dass** das Nahtelement (200) sich durch eine erste Durchgangsöffnung (11) der Mehrzahl von Durchgangsöffnungen (11, 12, 13, 14, 15, 16) von der Rückseite (100b) zur Vorderseite (100a) der Aufhängungsplatte (100) erstreckt, mit dem ersten Ende (201a) voran, insbesondere derart, dass der einstellbare erste Aufhängungsabschnitt (231) auf der Rückseite (100b) der Aufhängungsplatte (100) gebildet ist, und wobei vorzugsweise das Nahtelement (200) sich durch eine zweite Durchgangsöffnung (12) der Mehrzahl von Durchgangsöffnungen (11, 12, 13, 14, 15, 16) von der Vorderseite (100a) zur Rückseite (100b) der Aufhängungsplatte (100) erstreckt, mit dem ersten Ende (201a) voran, insbesondere derart, dass die erste Verriegelungsschlaufe (41) auf der Vorderseite (100a) der Aufhängungsplatte (100) gebildet ist, wobei das Nahtelement (200) sich ferner durch eine dritte Durchgangsöffnung (13) der Mehrzahl von Durchgangsöffnungen (11, 12, 13, 14, 15, 16) von der Rückseite (100b) zur Vorderseite (100a) der Aufhängungsplatte (100) erstreckt, mit dem ersten Ende (201a) voran, wobei insbesondere der erste Endabschnitt (201) des Nahtelements (200) sich ferner durch die erste Verriegelungsschlaufe (41) erstreckt, um den ersten Endabschnitt (201) mittels der ersten Verriegelungsschlaufe (41) mit einer Verriegelungskraft (F_{L}) an die Vorderseite (100a) der Aufhängungsplatte (100) zu klemmen, und wobei vorzugsweise das Nahtelement (200) sich ferner durch eine vierte Durchgangsöffnung (14) der Mehrzahl von Durchgangsöffnungen (11, 12, 13, 14, 15, 16) von der Vorderseite (100a) zur Rückseite (100b) der Aufhängungsplatte (100) erstreckt, mit dem ersten Ende (201a) voran, und wobei vorzugsweise das Nahtelement (200) sich ferner durch eine vierte Durchgangsöffnung (14) der Mehrzahl von Durchgangsöffnungen (11, 12, 13, 14, 15, 16) von der Rückseite (100b) zur Vorderseite (100a) der Aufhängungsplatte (100) erstreckt, mit dem zweiten Ende (202a) voran, insbesondere derart, dass das Nahtelement (200) einen zweiten Aufhängungsabschnitt (232) zwischen dem Verbindungsabschnitt (240) und der Aufhängungsplatte (100) bildet, wobei das Nahtelement (200) sich ferner durch eine fünfte Durchgangsöffnung (15) der Mehrzahl von Durchgangsöffnungen (11, 12, 13, 14, 15, 16) von der Vorderseite (100a) zur Rückseite (100b) der Aufhängungsplatte (100) erstreckt, mit dem zweiten Ende (202a) voran, insbesondere derart, dass eine zweite Verriegelungsschlaufe (42) gebildet wird, wobei das Nahtelement (200) sich ferner durch eine sechste Durchgangsöffnung (16) der Mehrzahl von Durchgangsöffnungen (11, 12, 13, 14, 15, 16) von der Rückseite (100b) zur Vorderseite (100a) der Aufhängungsplatte (100) erstreckt, mit dem zweiten Ende (202a) voran, wobei insbesondere der zweite Endabschnitt (202) des Nahtelements (200) sich ferner durch die zweite Verriegelungsschlaufe (42) erstreckt, um den zweiten Endabschnitt (202) des Nahtelements (200) mittels der zweiten Verriegelungsschlaufe (42) mit einer Verriegelungskraft (F_{L}) an die Vorderseite (100a) der Aufhängungsplatte (100) zu klemmen, und wobei vorzugsweise das Nahtelement (200) einen einstellbaren zweiten Aufhängungsabschnitt (232) zwischen dem Verbindungsabschnitt (240) und der Aufhängungsplatte (100) umfasst, wobei der zweite Aufhängungsabschnitt (232) dazu ausgebildet ist, durch Ziehen am zweiten Endabschnitt (202) des Nahtelements (200) verkürzt zu werden, wobei insbesondere der zweite Endabschnitt (202) aus der zweiten Verriegelungsschlaufe (42) herausragt.

10. Medizinisches Implantat (6), umfassend die implantierbare Aufhängevorrichtung (1) nach einem der Ansprüche 1 bis 9 und ein medizinisches Textil (500), insbesondere umfassend oder bestehend aus einem flachen Material, wobei der Verbindungsabschnitt (240) des Nahtelements (200) mit dem medizinischen Textil (500) verbunden ist, wobei das medizinische Textil (500) dazu ausgebildet ist, mit dem Implantat (G) verbunden zu werden.

11. Medizinisches Implantat (6) nach Anspruch 10, **dadurch gekennzeichnet, dass** der Verbindungsabschnitt (240) mittels Stichen (S) mit dem medizinischen Textil (500) verbunden ist.

12. Medizinisches Implantat (6) nach einem der Ansprüche 10 bis 11, **dadurch gekennzeichnet, dass** das medizinische Textil (500) eine röhrenförmige Gestalt aufweist, wobei das medizinische Textil (500) eine Innenoberfläche umfasst, die dazu ausgebildet ist, mit dem Implantat (G) verbunden zu werden.

## Revendications

1. Dispositif de suspension implantable (1) pour fixer un implant flexible allongé (G) dans une position souhaitée, comprenant : une plaque de suspension (100) et un élément de suture (200) s'engageant avec la plaque de suspension (100), dans lequel l'élément de suture (200) comprend une première section d'extrémité libre (201) s'étendant de ladite plaque de suspension (100) à une première extrémité (201a) dudit élément de suture (200) et une seconde section d'extrémité (202) s'étendant de ladite plaque de suspension (100) à une seconde extrémité (202a) dudit élément de suture (200), dans lequel la seconde section d'extrémité (202) comprend une section de connexion (240) configurée pour être connectée audit implant (G) ou à un textile médical (500), dans lequel ledit élément de suture (200) comprend ou est constitué d'une pluralité de fibres (203), dans lequel les fibres (203) sont séparées l'une de l'autre dans la section de connexion (240), **caractérisé en ce que** ladite section de connexion (240) forme une structure de type éventail s'étendant dans un plan parallèle à une direction de prolongement de l'élément de suture (200).

2. Dispositif de suspension implantable (1) selon la revendication 1, **caractérisé en ce que** ladite section de connexion (240) est positionnée à la seconde extrémité (202a) de l'élément de suture (200).

3. Dispositif de suspension implantable (1) selon la revendication 1 ou 2, **caractérisé en ce que** l'élément de suture (200) comprend une aiguille (250) reliée à l'élément de suture (200), notamment à la première extrémité (201a) ou la seconde extrémité (202a).

4. Dispositif de suspension implantable (1) selon une des revendications précédentes, **caractérisé en ce que** l'élément de suture (200) forme une première boucle de verrouillage (41) pour presser la première section d'extrémité (201) de l'élément de suture (200) contre la plaque de suspension (100) et verrouiller de ce fait la première section d'extrémité (201) par rapport à la plaque de suspension (100).

5. Dispositif de suspension implantable (1) selon une des revendications précédentes, **caractérisé en ce que** la seconde section d'extrémité (202) comprend une première section de suspension ajustable (231) entre ladite plaque de suspension (100) et ladite section de connexion (240), dans lequel la première section de suspension (231) est configurée pour être raccourcie en tirant sur la première section d'extrémité (201) de l'élément de suture (200), dans lequel ladite première section d'extrémité (201) fait notamment saillie hors de ladite première boucle de verrouillage (41).

6. Dispositif de suspension implantable (1) selon une des revendications précédentes, **caractérisé en ce que** la plaque de suspension (100) comprend un côté avant (100a) et un côté arrière (100b), lequel côté arrière (100b) est détourné du côté avant (100a).

7. Dispositif de suspension implantable (1) selon la revendication 6, **caractérisé en ce que** la plaque de suspension (100) comprend une pluralité d'orifices traversants (11, 12, 13, 14, 15, 16), chaque orifice traversant (11, 12, 13, 14, 15, 16) s'étendant du côté avant (100a) au côté arrière (100b) de ladite plaque de suspension (100), dans lequel ledit élément de suture (200) s'étend à travers lesdits orifices traversants (11, 12, 13, 14, 15, 16).

8. Dispositif de suspension implantable (1) selon la revendication 7, **caractérisé en ce que** ladite pluralité d'orifices traversants (11, 12, 13, 14, 15, 16) est formée d'au moins trois orifices traversants (11, 12, 13), notamment 3, 4 ou 6 orifices traversants.

9. Dispositif de suspension implantable (1) selon la revendication 7 ou 8, **caractérisé en ce que** l'élément de suture (200) s'étend à travers un premier orifice traversant (11) de ladite pluralité d'orifices traversants (11, 12, 13, 14, 15, 16) du côté arrière (100b) au côté avant (100a) de la plaque de suspension (100), avec la première extrémité (201a) à l'avant, notamment de sorte que la première section de suspension ajustable (231) est formée sur le côté arrière (100b) de la plaque de suspension (100), et dans lequel l'élément de suture (200) s'étend de préférence à travers un deuxième orifice traversant (12) de ladite pluralité d'orifices traversants du côté avant (100a) au côté arrière (100b) de la plaque de suspension (100), avec la première extrémité (201a) à l'avant, notamment de sorte que la première boucle de verrouillage (41) est formée sur le côté avant (100a) de la plaque de suspension (100), dans lequel l'élément de suture (200) s'étend en outre à travers un troisième orifice traversant (13) de ladite pluralité d'orifices traversants (11, 12, 13, 14, 15, 16) du côté arrière (100b) au côté avant (100a) de la plaque de suspension (100) avec la première extrémité (201a) à l'avant, dans lequel la première section d'extrémité (201) de l'élément de suture (200) s'étend en outre notamment à travers la première boucle de verrouillage (41) pour serrer la première section d'extrémité (201) sur le côté avant (100a) de la plaque de suspension (100) au moyen de la première boucle de verrouillage (41) avec une force de verrouillage (F_{L}), et dans lequel l'élément de suture (200) s'étend en outre de préférence à travers un quatrième orifice traversant (14) de ladite pluralité d'orifices traversants du côté avant (100a) au côté arrière (100b) de la plaque de suspension (100) avec la première extrémité (201a) à l'avant, et dans lequel l'élément de suture (200) s'étend en outre de préférence à travers un quatrième orifice traversant (14) de ladite pluralité d'orifices traversants (11, 12, 13, 14, 15, 16) du côté arrière (100b) au côté avant (100a) de la plaque de suspension (100) avec la seconde extrémité (202a) à l'avant, notamment de sorte que l'élément de suture (200) forme une seconde section de suspension (232) entre ladite section de connexion (240) et ladite plaque de suspension (100), et dans lequel ledit élément de suture (200) s'étend en outre à travers un cinquième orifice traversant (15) de ladite pluralité d'orifices traversants (11, 12, 13, 14, 15, 16) du côté avant (100a) au côté arrière (100b) de la plaque de suspension (100) avec la seconde extrémité (202a) à l'avant, notamment de sorte qu'une seconde boucle de verrouillage (42) est formée, dans lequel l'élément de suture (200) s'étend en outre à travers un sixième orifice traversant (16) de ladite pluralité d'orifices traversants (11, 12, 13, 14, 15, 16) du côté arrière (100b) au côté avant (100a) de la plaque de suspension (100) avec la seconde extrémité (202a) à l'avant, dans lequel la seconde section d'extrémité (202) de l'élément de suture (200) s'étend en outre notamment à travers ladite seconde boucle de verrouillage (42) pour serrer la seconde section d'extrémité (202) de l'élément de suture (200) sur le côté avant (100a) de la plaque de suspension (100) au moyen de la seconde boucle de verrouillage (42) avec une force de verrouillage (F_{L}), et dans lequel l'élément de suture (200) comprend de préférence une seconde section de suspension ajustable (232) entre ladite section de connexion (240) et ladite plaque de suspension (100), dans lequel la seconde section de suspension (232) est configurée pour être raccourcie en tirant sur la seconde section d'extrémité (202) de l'élément de suture (200), dans lequel ladite seconde section d'extrémité (202) fait notamment saillie hors de ladite seconde boucle de verrouillage (42).

10. Implant médical (6) comprenant le dispositif de suspension implantable (1) selon une des revendications 1 à 9 et un textile médical (500), comprenant ou étant constitué notamment d'un matériau de feutre, dans lequel la section de connexion (240) de l'élément de suture (200) est connectée au textile médical (500), dans lequel le textile médical (500) est configuré pour être connecté audit implant (G).

11. Implant médical (6) selon la revendication 10, **caractérisé en ce que** la section de connexion (240) est connectée au textile médical (500) par des points (S).

12. Implant médical (6) selon une des revendications 10 à 11, **caractérisé en ce que** le textile médical (500) comprend une forme tubulaire, dans lequel le textile médical (500) comprend une surface interne configurée pour être connectée audit implant (G).
